# FASCICULE DE BREVET EUROPEEN

(11) **EP 0 430 806 B1**
(45) Date de publication et mention de la délivrance du brevet: **08.06.1994**
(21) Numéro de dépôt: 90403392.5
(22) Date de dépôt: 29.11.1990
(51) Int. Cl.: C07C 43/313, C07C 251/30, C07D 295/06, C07D 317/16, C07C 41/50

(54) **Procédé de préparation d'halogénoacétals à partir d'énamines**
Verfahren zur Herstellung von Halogenacetalen aus Enaminen
Process for the preparation of haloketals from enamines

(30) Priorité: 01.12.1989 FR 8915868
(43) Date de publication de la demande: 05.06.1991
(73) Titulaire: RHONE-POULENC NUTRITION ANIMALE, 03600 Commentry (FR)
(72) Inventeur: Chabardes, Pierre, F-69110 Sainte-Foy Les Lyon (FR); Duhamel, Lucette, F-76130 Mont-Saint-Aignan (FR); Duhamel, Pierre, F-76130 Mont-Saint-Aignan (FR); Guillemont, Jérômes, F-27210 Beuzeville (FR); Poirier, Jean-Marie, F-76160 Saint-Martin du Vivier (FR)
(74) Mandataire: Le Pennec, Magali

(56) Documents cités:
- DE-A- 2 708 281
- DE-A- 2 708 304
- US-A- 3 428 633
- Bull. Soc. Chim. Fr., 1964. pages 1205, 1206
- Bull. Chem. Soc. Jpn. Vol.58, pages 2427, 2428;
- Chem. Lett. 1982, pages 1307, 1308;
- Tet. Lett. 1972, pages 1997-2000

## Description

La présente invention concerne un nouveau procédé de préparation d'halogénoacétals d'aldéhydes éthyléniques de formule générale : 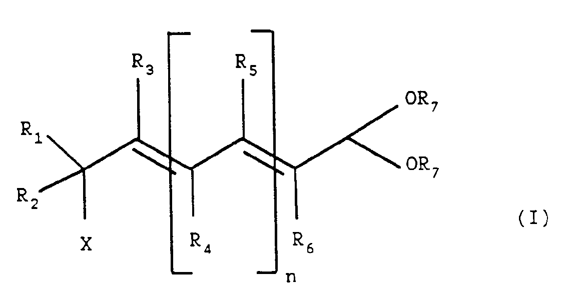
dans laquelle :
- X représente un atome d'halogène choisi parmi les atomes de chlore, de brome et d'iode
- R₁, R₂ , R₃, R₄, R₅ et R₆ identiques ou différents, représentent un atome d'hydrogène ou un radical alkyle contenant 1 à 6 atomes de carbone en chaîne droite ou ramifiée, en particulier un radical méthyle ou éthyle, ou alcényle contenant 3 à 6 atomes de carbone en chaîne droite ou ramifiée et dont la double liaison est en position autre que 1-2.
- n est égal à 0, 1, 2, 3 ou 4, étant entendu que lorsque n est supérieur à 1, les différents symboles R₄ et R₅ peuvent être identiques ou différents : les symboles R₇ représentent chacun un radical alkyle contenant 1 à 6 atomes de carbone en chaîne droite ou ramifiée, en particulier un radical méthyle ou éthyle, ou forment ensemble un radical alkylène R′₇ contenant 2 à 6 atomes, de carbone en chaîne droite ou ramifiée, en particulier un radical -CH₂-CH₂-, et éventuellement substitué par un radical hydroxyle ou alkyloxy contenant 1 à 4 atomes de carbone.

Il est précisé que le terme halogène tel qu'utilisé ci-après englobe uniquement chlore, brome et iode.

Les halogénoacétals d'aldéhydes éthyléniques sont des composés organiques particulièrement utiles comme intermédiaires en synthèse organique. C'est ainsi qu'ils peuvent être utilisés pour introduire un motif aldéhydique α,β-éthylénique sur un reste mono ou polyénique par réaction avec une sulfone polyénique en présence d'un agent alcalin selon le procédé général décrit dans le brevet anglais 1396622, la sulfone résultant de cette condensation étant ensuite désulfonée avec formation d'une double liaison supplémentaire.

Il est connu que les γ-halogénoacétals d'aldéhydes α,β-éthyléniques peuvent être préparés par halogénoalkylation d'un alkyloxy-1 diène-1,3 par action d'un N-halogénosuccinimide en présence d'un alcool suivant le procédé décrit par S.M. MAKIN et coll., J. Gen. Chem. URSS, 32, 1088 (1962). Mais ce procédé a pour inconvénient la difficulté d'accès aux éthers diéthyléniques de départ ; ceux-ci sont en effet généralement préparés par traitement d'acétals d'aldéhydes α, β- ou β, γ-éthyléniques à haute température en présence de catalyseurs, les matières premières utilisées étant elles-mêmes de synthèse difficile. Bien que la méthode de MAKIN soit également applicable à la synthèse d'ω-halogénoacétals d'aldéhydes comportant un système de doubles liaisons conjuguées, la préparation de tels produits par cette méthode présente de très grandes difficultés du fait de l'accès difficile aux matières premières nécessaires.

Il est aussi connu dans le brevet US 4100201 de préparer les ω-halogénoacétals d'aldéhydes éthyléniques par halogénation d'un ester de préférence acétique correspondant à l'aldéhyde éthylénique. Cet ester est préparé selon l'article de M.J. MAGEMEYER paru dans Ind. Eng. Chem. 41, 2920 (1949) par échange entre l'aldéhyde et l'acétate d'isopropényle. Lors de la regénération de l'aldéhyde après hydrolyse de la fonction ester l'acide acétique est libéré ce qui ne permet pas de régénerer la matière première couteuse qu'est l'acétate d'isopropényle. Ce procédé est donc, comme le précédent, non économique.

Il est encore connu d'après le brevet US4087465 de préparer les ω-halogénoacétals d'aldéhydes polyéniques par réaction d'un cation halogène sur un énoxysilane. L'énoxysilane est un composé difficilement accessible d'un point de vue industriel et par là même une matière première coûteuse.

Le procédé selon l'invention permet d'accéder aux halogénoacétals d'aldéhydes insaturés avec de bons rendements à partir de matières premières accessibles, peu couteuses et facilement recyclables.

Il a en effet été trouvé que l'on peut préparer les halogénoacétals d'aldéhydes éthyléniques de formule générale (I) par réaction d'un agent d'halogénation choisi parmi les cations halogènes Cl⁺, Br⁺ et I⁺ sur une énamine de formule générale : 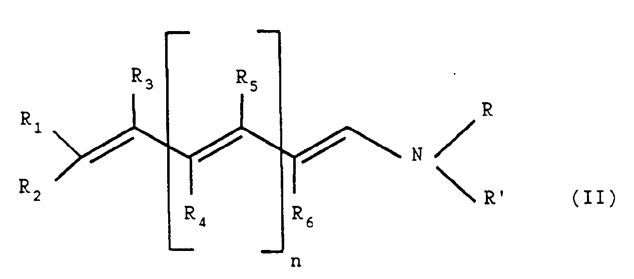
dans laquelle R₁, R₂, R₃, R₄, R₅ R₆ et n sont définis comme précédemment et R et R′ représentent des radicaux alkyle contenant 1 à 6 atomes de carbone en chaîne droite ou ramifiée ou un radical alkylène ou alkenylène contenant éventuellement des hétéroatomes choisis parmi l'oxygène, le soufre et l'azote, en particulier ils peuvent former un cycle pipéridinique ou morpholinique, puis, après action de l'eau, réaction du produit obtenu sur un alcool primaire ou secondaire de formule générale R₇OH dans laquelle R₇ est défini comme précédemment ou sur un glycol de formule générale HO-R′₇-OH dans laquelle R′₇ est défini comme précédemment ou encore sur un orthoformiate de formule HC(OR₇)₃ ou mixte de formule (O₂R₇′)CH-(OR₇) obtenu par condensation d'un orthoformiate HC(OR₇)₃ et d'un glycol de formule HOR₇′OH.

Les cations halogènes sont connus depuis de nombreuses années, en particulier par J. AROTSKY et M.C.R. SYMONS, Quart. Rev. 16282 (1962) ; il est également connu que ces cations halogènes peuvent être mis en évidence par différentes méthodes, telles que la mesure de la conductivité et la spectrométrie de masse par exemple. De nombreux produits sont connus pour être la source de tels cations halogènes [Peter B.D. de La Marre, "Electrophilic Halogenation", Cambridge Chemistry Texts, 1976]. Une première classe de produits utilisables comme source de cations halogènes est constituée par les halogènes eux mêmes et les produits dans lesquels un atome d'halogène est lié par liaison covalente à un atome d'azote d'oxygène, de phosphore ou de soufre ; à titre d'exemple on peut citer en particulier les hypohalogénites alcalins, les hypohalogénites organiques, les N-halogénoamines, les N-halogénoamides, les N-halogéno-carbo-imides, les N-halogéno-sulfo-imides, les N-halogéno-carbo-sulfo-imides les N-halogéno-hydantoines, les N-halogéno-triazoles et N-benzo-triazoles, les oxychlorures de phosphore, le chlorure de thionyle. Une seconde classe de produits utilisables comme source de cations halogènes est constituée par les composés résultant de l'addition d'halogène moléculaire sur un halogénure d'ammonium quaternaire aliphatique, aromatique ou cyclique ou sur un halogénure aromatique. Une troisième classe de produits utilisables comme source de cations halogènes est constituée par les complexes formés par action d'un halogène moléculaire sur un amide aliphatique ou cyclique.

Peuvent être plus spécialement cités comme sources de cations halogènes, les hypohalogénites alcalins, les hypohalogénites organiques, en particulier les hypohalogénites d'alcools aliphatiques tertiaires saturés contenant jusqu'à 13 atomes de carbone, les N-chloro et N-bromo-succinimides, les N-bromo et N-chloro polymaléimides, les N-bromo et N-chloro caprolactames, les dichloro-1,3 et dibromo-1,3 diméthyl-5,5 hydantoines, les N-bromo et N-chloro saccharines, le chlorobenzotriazole, le N-bromo acétamide, la bromourée, la chloramine, le perbromure de phényl-triméthylammonium, l'iodure de tétrachlorotétra-n-butylammonium, l'iodure de dichloro tétra-n-butylammonium, le tribromure de tétra-n-butylammonium, le perbromure de pyridinium, le dichlorure d'iodobenzène, les oxychlorures de phosphore, le chlorure de thionyle, les complexes fournis par action du chlore, du brome ou de l'iode sur le diméthylformamide, le diméthylacétamide et la N-méthylpyrrolidone.

D'une manière générale, il est suffisant de faire réagir un cation halogène par mole d'énamine de formule générale (II), c'est-à-dire d'utiliser la quantité de produit générateur de cation halogène nécessaire pour fournir un cation halogène par mole d'énamine de formule générale (II), un excès de l'un ou de l'autre de ces réactifs pouvant toutefois être utilisé sans inconvénient. Après la réaction entre le cation halogène et l'énamine de formule générale (II), on ajoute de l'eau, puis un excès de l'alcool de formule générale R₇OH, d'orthoformiate HC(OR₇)₃ ou mixte ou encore du glycol de formule générale HO-R′₇-OH. Cette réaction permet de réaliser la transformation de l'énamine de formule générale (II) en halogénoacétal de formule générale (I), dans le même milieu réactionnel ("one pot synthesis"), sans isoler les produits intermédiaires. La température de la réaction n'est pas critique et peut être comprise par exemple entre -70 et +80°C, et de préférence entre -60 et +20°C afin d'éviter une décomposition appréciable des produits.

Lorsque l'on utilise comme source de cation halogène des halogènes moléculaires ou des composés dans lesquels l'atome d'halogène est lié à un atome d'azote ou d'oxygène ou un composé résultant de l'addition d'un halogène moléculaire sur un halogénure d'ammonium quaternaire aliphatique aromatique ou cyclique ou sur un halogénure aromatique, on opère généralement à une température comprise entre -70°C et +80°C selon la stabilité du produit utilisé comme source de cation halogène et particulièrement entre -70 et +30°C. Afin d'accélérer la vitesse de la réaction d'acétalisation, il est avantageux d'opérer en présence d'une quantité catalytique d'un acide minéral ou organique connu comme catalyseur d'acétalisation, tel que les acides chlorhydrique, sulfurique et méthanesulfonique ; cet acide peut être introduit dans le mélange réactionnel après réaction du produit générateur de cation halogène sur l'énamine de formule générale (II).

Lorsque l'on condense l'halogène cationique dont la source a pour formule AX sur l'énamine de formule (II) il se forme intermédiairement un sel d'iminium de formule générale (III) qui est un produit nouveau. 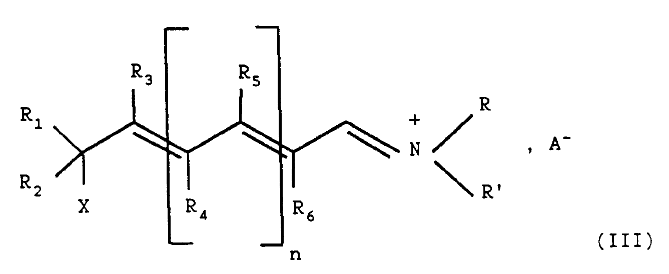
formule dans laquelle X, R₁, R₂, R₃, R₄, R₅, R₆, R, R′ et n ont la même signification que dans la formule (I) et (II), A⁻ représente l'anion lié au cation halogène dans la source de cation halogène. A⁻peut ainsi représenter : un halogène X si la source de cation halogène est l'halogène moléculaire, un groupe par exemple oxydichlorure de phosphore (POCl₂⁻), un groupe par exemple monochlorure de thionyle (SOCl⁻).

Il peut également se former à côté du produit (III) un produit isomère (IIIa) de formule suivante : 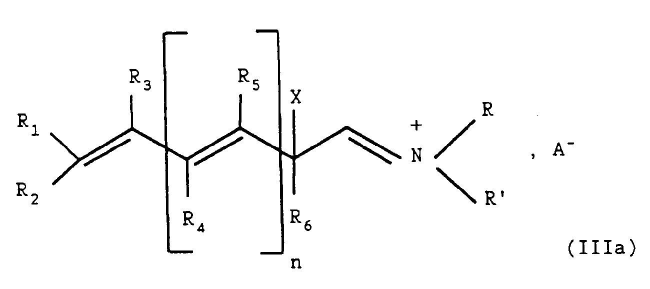
qui sera facilement converti en produit de formule (III) simplement par augmentation de la température du milieu réactionnel.

Les énamines de formule (II) utilisées comme matières premières sont préparées selon S.HUNIG et H. KAHANEK, Chem. Ber. 1957, 90, 238, N. F. FIRRELL, P.W. HICKMOTT et B.J. HOPKINS J.C.s. (B) 1971, 351, E.DEMOLE, C. DEMOLE ET P. ENGGIST Helv. Chim. Act. 1976, 59, 737 par mise en contact de l'amine de formule 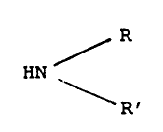
avec l'aldéhyde polyénique en présence d'un déshydratant dans un solvant aliphatique ou aromatique inerte vis-à-vis des réactifs. Les réactifs sont mis en présence à la température ambiante. Après réaction, le déshydradant est éliminé, le solvant est éliminé par distillation et l'énamine est obtenue par distillation.

Après halogénation et acétalisation on récupère d'une part l'halogénoacétal éthylénique de formule (I) et l'amine peut être facilement recyclée.

Les produits obtenus de formule (I) (n = 0) sont utilisés notamment pour la synthèse de la vitamine E ou du rétinal par condensation directement avec une sulfone contenant 15 atomes de carbone selon le procédé décrit dans le brevet GB 1396622 ou par passage par un intermédiaire phosphonique IV qui est ensuite condensé en présence d'une base sur le β-ionylidène acétaldéhyde. L'intermédiaire phosphonique (IV) est préparé par condensation du dérivé de formule (I), (n = 0) avec un alkylphosphite.

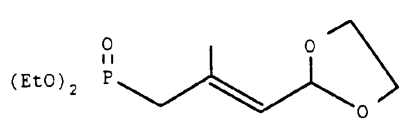

Le procédé selon la présente invention convient particulièrement bien pour la préparation du dioxolanne du méthyl-3 bromo-4 butène-2 al-1 à partir du morpholino-1 méthyl-3 butadiène-1,3.

Les exemples suivants, donnés à titre non limitatif, montrent comment le procédé selon la présente invention peut être mis en pratique.

### EXEMPLE 1

### Préparation du méthyl 1-3 morpholino-1 butadiène

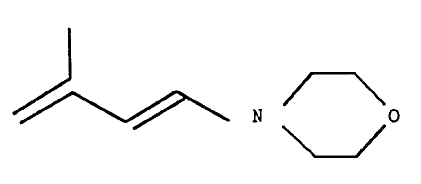

Sous atmosphère inerte, dans un erlenmeyer de 100 ml muni d'une ampoule à brome, on introduit 43,5 mmol de carbonate de potassium anhydre (6 g) et 308 mmol de morpholine (26,8 g). Une fois le milieu réactionnel refroidi à 0°C, on ajoute goutte à goutte 150 mmol de prénal dans 20 ml de toluène. On laisse remonter la température à 20°C et l'on poursuit l'agitation pendant 4 heures. On sépare le carbonate de potassium par filtration. On élimine le toluène à la pression atmosphérique ce qui permet le cracking de l'aminal. Sous pression réduite on distille tout d'abord la morpholine en excès, puis la diénamine attendue (Eb 108-110°C sous 20mm de Hg).

### EXEMPLE 2

### Préparation du bromo-4 méthyl-3 butène-2 al.

A 10 mmol de diénamine (1,53g) de l'exemple 1 en solution dans 100 ml d'éther anhydre, on ajoute à une température inférieure à -60°C une solution de 10 mmol de brome (1,6 g) dans 50 ml d'éther anhydre préalablement refroidie à -70°C. Après un quart d'heure d'agitation à -70°C, on remonte en cinq minutes à +35°C. Le reflux de l'éther est maintenu pendant 15 minutes.

Pour caractériser le sel d'iminium ω-bromé, le précipité jaune est filtré à l'abri de l'humidité, puis séché au dessicateur 3 heures à température ambiante en présence de P₄O₁₀. Le spectre RMN proton effectué dans l'acétonitrile deutéré a permis de caractériser le sel d'iminium ω-bromé de formule : 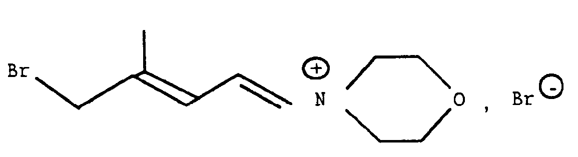
Sa formation est quantitative.
RMN ¹H,CD₃CN : isomère présent à 25 % :
9,5 (d,1H,J=11Hz) ; 6,7 (d,1H,J=11Hz) ; 4,7 (s,2H) ; 4-3,7(m,8H) ; 2,2 (s,3H).
RMN ¹H,CD₃CN, : isomère présent à 75 % ; 9,1 (d,1H,J=11Hz); 7,1 (d,1H,J=11Hz) ; 4,3 (s,2H) ; 4-3,7(m,8H) ; 2,3 (s,3H).

On procède dans le même réacteur à l'hydrolyse du sel d'iminium par 11 équivalents d'eau (2 ml) distillée. Le milieu réactionnel est agité vigoureusement à température ambiante pendant deux heures.

On vérifie la formation du prénal ω-bromé en l'isolant (formule A) :
Eluant : éther/éther de pétrole : 10/100.
Rdt : 70 %. IR : 1670 (ν C=0) ; 1640 (ν C=C).

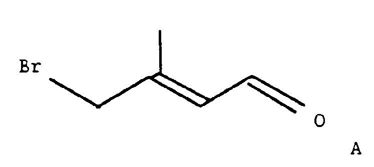

Si, dans le réacteur, on procède à l'hydrolyse par 111 équivalents d'eau (2 ml) à -70°C, 30 minutes après l'addition du brome, on isole un produit brut (Rdt : 88%), constitué d'aldéhydes isomères A et B (A/B = 63/37)

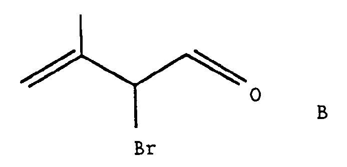

### EXEMPLE 3

### Préparation du dioxolanne du bromo-4 méthyl-3 butène-2 al

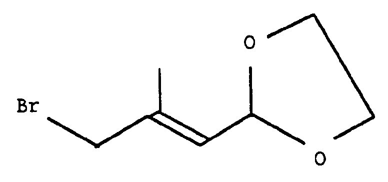

Sur le milieu réactionnel obtenu selon l'exemple 2, on introduit successivement 20ml d'un orthoformiate mixte l'éthylènedioxyméthoxy méthane (19 eq) et 4 gouttes d'une solution de chlorure d'hydrogène/éthylène glycol à 12% en poids. Il est nécessaire de maintenir le mélange réactionnel sous agitation pendant une heure avant d'observer en CCM la disparition complète de l'aldéhyde.

La neutralisation du milieu est réalisée par addition de méthylate de sodium solide (environ 100 mg). On sèche sur sulfate de magnésium et on concentre. On récupère 1,5 g de produit brut.Après purification par chromatographie éclair sur support de silice, on isole 49 % d'acétal ω-bromé.

### EXEMPLE 4

### Préparation du phosphonate du dioxolanne du méthyl-3 butène-2 al

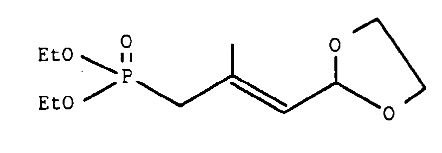

Dans un bicol de 25 ml, muni d'un thermomètre et d'un réfrigérant, on introduit successivement 0,83 g (5 mmol) de triéthyle phosphite dans 5 ml de toluène sec et 1 g (4,83 mmol) de bromo dioxolanne de l'exemple 3 dans 10 ml de toluène.

Le milieu réactionnel est chauffé au reflux du toluène durant 15 heures.

Après disparition totale en CCM du spot de l'acétal bromé et élimination du solvant, le phosphonate est purifié par distillation sous pression réduite (point d'ébullition : 110-120°C/0,3 mmHg), rendement : 80 %.

### EXEMPLE 5

### Préparation du rétinal

Sous argon, dans un bicol de 25 ml, on introduit 0,7 mmol de phosphonate de l'exemple 4 (0,185 g) en solution dans 10 ml de THF. On ajoute par petites fractions à -70°C, 1 mmol de tertiobutylate de potassium (0,112 g). On poursuit l'agitation 90 minutes à -70°C. On ajoute ensuite, 0,5 mmol de β-ionylidène acétaldéhyde (0,109 g) en solution dans un ml de THF. La température est maintenue 15 minutes à -70°C puis, 1 heure à -20°C.

L'hydrolyse du milieu réactionnel est effectuée à -20°C avec une solution d'acide chlorhydrique 3 N(5ml). Après un quart d'heure d'agitation, on extrait la phase aqueuse à l'éther (5 fois 15 ml), sèche sur sulfate de magnésium et concentre. Le produit brut est purifié par chromatographie éclair (éluant éther/éther de pétrole 10/100). Le rendement en rétinal est de 66 %.

## Revendications

1. Procédé de préparation d'halogénoacétals d'aldéhydes éthyléniques de formule générale : 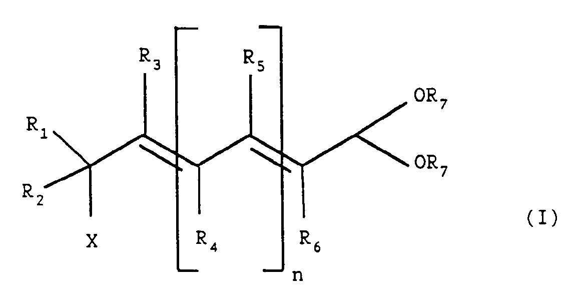 dans laquelle :
- X représente un atome d'halogene choisi parmi les atomes de chlore, de brome et d'iode
- R₁, R₂, R₃, R₄ , R₅, R₆ identiques ou différents, représentent un atome d'hydrogène ou un radical alkyle contenant 1 à 6 atomes de carbone en chaîne droite ou ramifiée, ou alcényle contenant 3 à 6 atomes de carbone en chaîne droite ou ramifiée et dont la double liaison est en position autre que 1-2.
- n est égal à 0, 1, 2, 3 ou 4, étant entendu que lorsque n est supérieur à 1, les différents symboles R₄ et R₅ peuvent être identiques ou différents.
- les symboles R₇ représentent chacun un radical alkyle contenant 1 à 6 atomes de carbone en chaîne droite ou ramifiée ou forment ensemble un radical alkylène R′₇ contenant 2 à 6 atomes de carbone en chaîne droite ou ramifiée et éventuellement substitué par un radical hydroxyle ou alkyloxy contenant 1 à 4 atomes de carbone caractérisé en ce que l'on fait réagir un cation halogène choisi parmi Cl⁺, Br⁺ et I⁺ sur une enamine de formule générale : 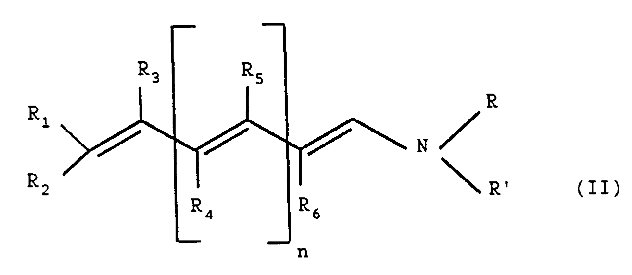 dans laquelle R₁, R₂, R₃, R₄, R₅, R₆ et n sont définis comme précédemment et R et R′ représentent des radicaux alkyle contenant 1 à 6 atomes de carbone en chaîne droite ou ramifiée ou un radical alkylène ou alkenylène contenant éventuellement des hétéroatomes choisis parmi l'oxygène, le soufre et l'azote, et en ce qu'on fait réagir le produit obtenu après hydrolyse avec un alcool primaire ou secondaire de formule générale R₇OH dans laquelle R₇ est défini comme précédemment ou sur un glycol de formule générale HO-R′₇-OH dans laquelle R′₇ est défini comme précédemment, ou un orthoformiate de formule générale HC(OR₇)₃ ou mixte.

2. Procédé selon la revendication 1 caractérisé en ce que la réaction entre le cation halogène et l'énamine de formule générale: 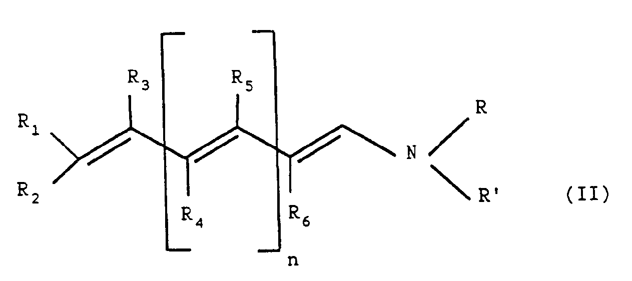 est suivie d'addition successives d'eau et d'alcool de formule générale R₇OH ou du glycol de formule générale HO-R′₇-OH ou d'orthoformiate de formule générale HC(OR₇)₃ ou mixte.

3. Procédé selon les revendications 1 ou 2 caractérisé en ce que la source de cation halogène XA est constituée par l'halogène moléculaire ou par un produit dans lequel un atome d'halogène est lié par liaison covalente à un atome d'azote, d'oxygène de phosphore ou de soufre.

4. Intermédiaires ioniques répondant à la formule générale suivante : 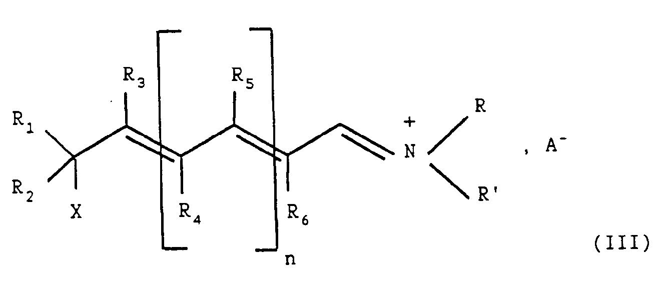 dans laquelle A⁻ représente l'anion lié au cation halogène dans la source AX de cation halogène, R₁ à R₆, R, R′, X et n ont la même signification que dans les formules (I) et (II).

5. Intermédiaires ioniques répondant à la formule générale suivante : 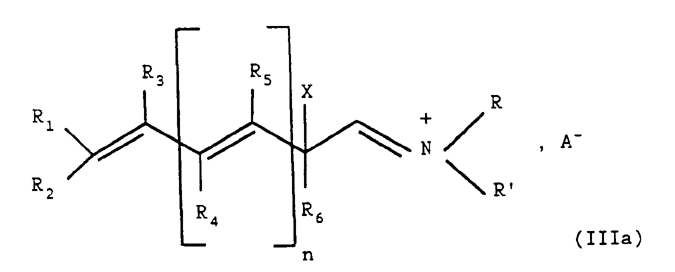 dans laquelle A-, R₁ à R₆, R, R′, X et n ont la même signification que dans les formules (I), (II), et (III).

## Patentansprüche

1. Verfahren zur Herstellung von Halogenacetalen ethylenischer Aldehyde mit folgender allgemeinen Formel: 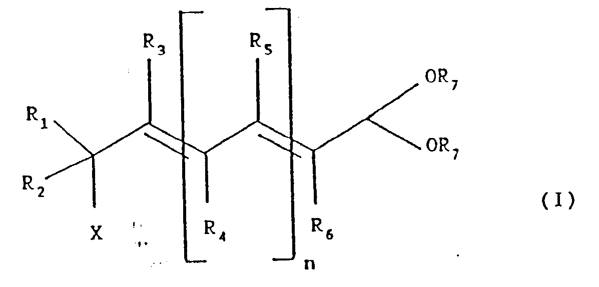 in der:
- X für ein Halogenatom steht, ausgewählt aus den Chlor-, Brom- und lodatomen,
- R₁, R₂, R₃, R₄, R₅, R₆, seien sie gleich oder verschieden, für ein Wasserstoffatom stehen oder für einen Alkylrest mit 1 bis 6 Kohlenstoffatomen in linearer oder verzweigter Kette, oder für einen Alkenylrest mit 3 bis 6 Kohlenstoffatomen in linearer oder verzweigter Kette, dessen Doppelbindung in einer anderen als in 1-2-Stellung liegt,
- n gleich 0, 1, 2, 3 oder 4 ist, wobei die verschiedenen Symbole R₄ und R₅ gleich oder verschieden sein können, wenn n größer als 1 ist,
- jedes der Symbole R₇ für einen Alkylrest mit 1 bis 6 Kohlenstoffatomen in linearer oder verzweigter Kette steht oder in der die Symbole R₇ zusammen einen Alkylenrest R′₇ bilden mit 2 bis 6 Kohlenstoffatomen in linearer oder verzweigter Kette, der gegebenenfalls substituiert ist mit einem Hydroxyl- oder Alkyloxyrest mit 1 bis 4 Kohlenstoffatomen, dadurch gekennzeichnet, daß man ein Halogenkation, ausgewählt aus Cl⁺, Br⁺ und I⁺, mit einem Enamin mit folgender allgemeinen Formel reagieren läßt: 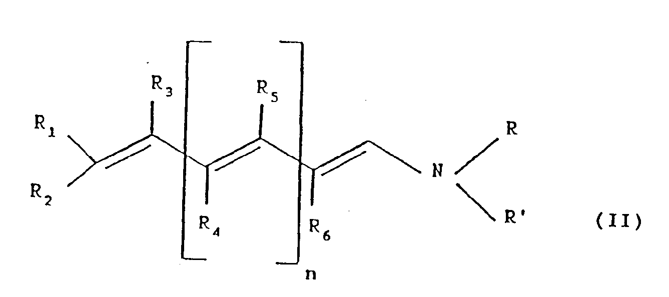 in der R₁, R₂, R₃, R₄, R₅, R₆ und n wie vorher definiert sind und R und R′ für Alkylreste mit 1 bis 6 Kohlenstoffatomen in linearer oder verzweigter Kette stehen oder für einen Alkylen- oder Alkenylenrest, der gegebenenfalls Heteroatome enthält, ausgewählt aus dem Sauerstoff, dem Schwefel und dem Stickstoff, und dadurch, daß man das nach Hydrolyse erhaltene Produkt mit einem primären oder sekundären Alkohol mit der allgemeinen Formel R₇OH reagieren läßt, in der R₇ wie vorher definiert ist, oder mit einem Glykol mit der allgemeinen Formel HO-R′₇-OH, in der R′₇ wie vorher definiert ist, oder mit einem Orthoformiat mit der allgemeinen Formel HC(OR₇)₃ oder einem gemischten.

2. Verfahren nach Anspruch 1, dadurch gekennzeichnet, daß die Reaktion zwischen dem Halogenkation und dem Enamin mit der allgemeinen Formel: 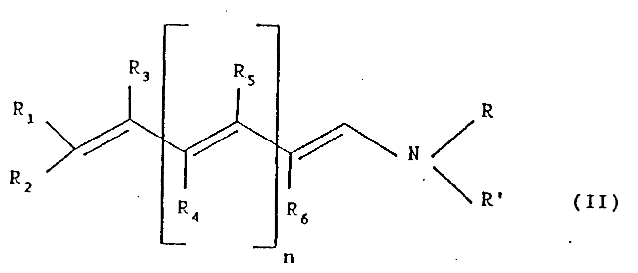 gefolgt ist von aufeinanderfolgenden Zugaben von Wasser und von Alkohol mit der allgemeinen Formel R₇OH oder von Glykol mit der allgemeinen Formel HO-R′₇-OH oder von Orthoformiat mit der allgemeinen Formel HC(OR₇)₃ oder einem gemischten.

3. Verfahren nach den Ansprüchen 1 oder 2, dadurch gekennzeichnet, daß der Ausgangstoff für das Halogenkation XA aus dem molekularen Halogen oder aus einem Produkt besteht, in dem ein Halogenatom mit einer kovalenten Bindung an ein Stickstoff-, Sauerstoff-, Phosphor- oder Schwefelatom gebunden ist.

4. Ionische Zwischenprodukte, die folgender allgemeinen Formel entsprechen: 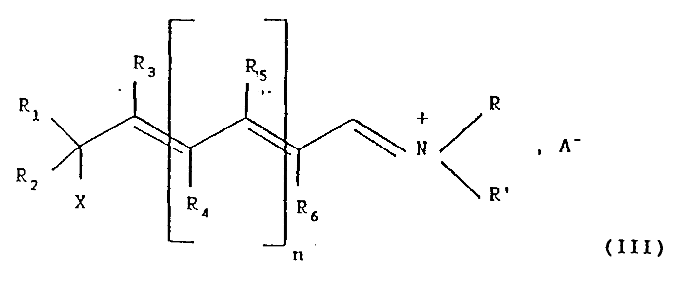 in der A⁻ für das Anion steht, das an das Halogenkation in dem Ausgangsstoff AX für das Halogenkation gebunden ist, R₁ bis R₆, R, R′, X und n die gleiche Bedeutung wie in den Formeln (I) und (II) haben.

5. Ionische Zwischenprodukte, die folgender allgemeiner Formel entsprechen: 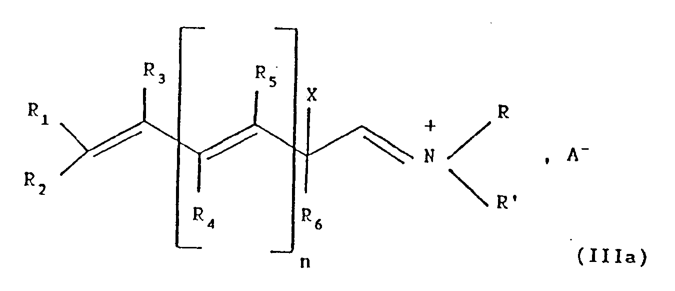 in der A⁻, R₁ bis R₆, R, R′, X und n die gleiche Bedeutung wie in den Formeln (I), (II) und (III) haben.

## Claims

1. Process for the preparation of halo acetals of ethylenic aldehydes of general formula: 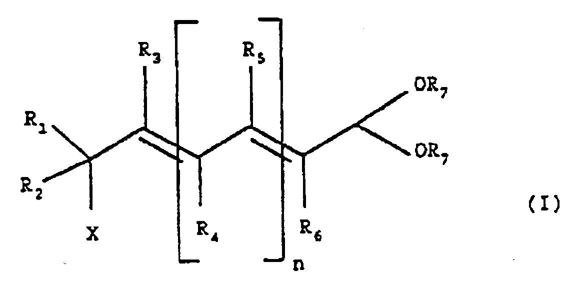 in which:
- X represents a halogen atom chosen from chlorine, bromine and iodine atoms
- R₁, R₂, R₃, R₄, R₅ and R₆, which may be identical or different, represent a hydrogen atom or an alkyl radical containing 1 to 6 carbon atoms in a straight or branched chain, or an alkenyl radical containing 3 to 6 carbon atoms in a straight or branched chain and in which the double bond is in a position other than 1-2.
- n is equal to 0, 1, 2, 3 or 4, it being understood that when n is greater than 1, the different symbols R₄ and R₅ may be identical or different.
- the symbols R₇ each represent an alkyl radical containing 1 to 6 carbon atoms in a straight or branched chain, or together form an alkylene radical R′₇ containing 2 to 6 carbon atoms in a straight or branched chain, and optionally substituted by a hydroxyl radial or an alkyloxy radical containing 1 to 4 carbon atoms, characterized in that a halogen cation chosen from Cl⁺, Br⁺ and I⁺ is reacted with an enamine of general formula: 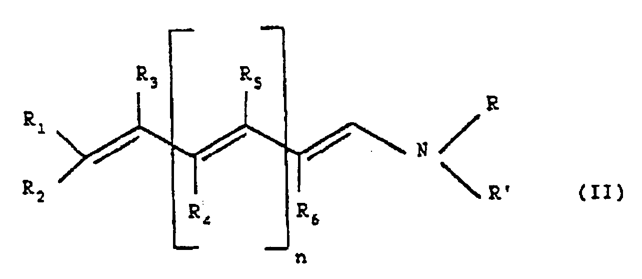 in which R₁, R₂, R₃, R₄, R₅, R₆ and n are defined as above and R and R′ represent alkyl radicals containing 1 to 6 carbon atoms in a straight or branched chain, or an alkylene or alkenylene radical optionally containing hetero atoms chosen from oxygen, sulphur and nitrogen, and in that the product obtained after hydrolysis is reacted with a primary or secondary alcohol of general formula R₇OH in which R₇ is defined as above, or with a glycol of general formula RO-R′₇-OH in which R′₇ is defined as above, or an orthoformate of general formula HC(OR₇)₃ or mixed orthoformate.

2. Process according to claim 1, characterized in that the reaction between the halogen cation and the enamine of general formula: 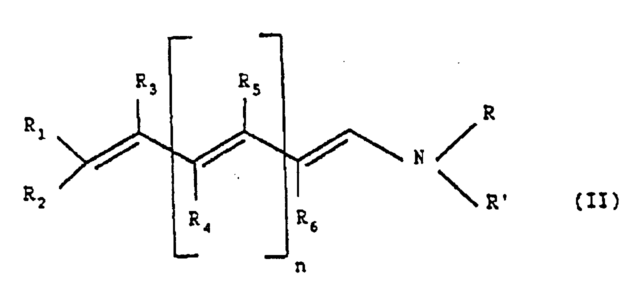 is followed by successive additions of water and of alcohol of general formula R₇OH, or of glycol of general formula HO-R′₇-OH, or of orthoformate of general formula HC(OR₇)₃ or mixed orthoformate.

3. Process according to claim 1 or 2, characterized in that the source of halogen cation XA consists of the molecular halogen or of a product in which the halogen atom is linked via a covalent bond to a nitrogen, oxygen, phosphorus or sulphur atom.

4. Ionic intermediates corresponding to the following general formula: 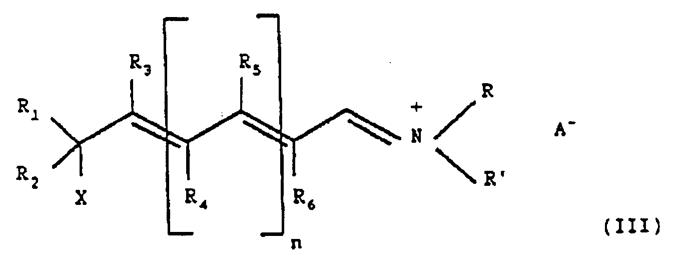 in which A⁻ represents the anion linked to the halogen cation in the source XA of halogen cation, and R₁ to R₆, R, R′, X and n have the same meaning as in the formulae (I) and (II).

5. Ionic intermediates corresponding to the following general formula: 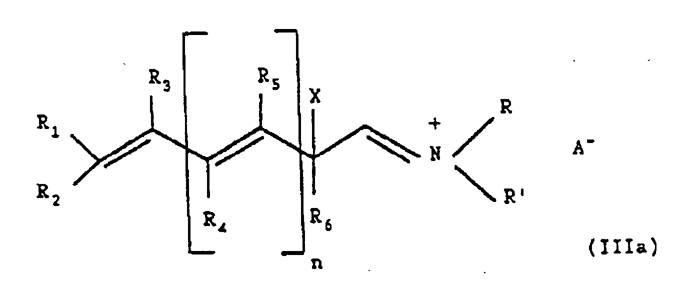 in which A⁻, R₁ to R₆, R, R′, X and n have the same meaning as in the formulae (I), (II) and (III).
